# EUROPÄISCHE PATENTSCHRIFT

(11) **EP 1 206 439 B1**
(45) Veröffentlichungstag und Bekanntmachung des Hinweises auf die Patenterteilung: **28.03.2007**
(21) Anmeldenummer: 00956304.0
(22) Anmeldetag: 26.07.2000
(51) Int. Cl.: C07C 67/36, C07C 69/06

(54) **VERFAHREN ZUR KONTINUIERLICHEN HERSTELLUNG VON METHYLFORMIAT**
METHOD FOR THE CONTINUOUS PRODUCTION OF METHYL FORMIATE
PROCEDE PERMETTANT DE PRODUIRE EN CONTINU DU FORMIATE DE METHYLE

(30) Priorität: 26.07.1999 DE 19935038
(43) Veröffentlichungstag der Anmeldung: 22.05.2002
(73) Patentinhaber: BASF Aktiengesellschaft, 67056 Ludwigshafen (DE)
(72) Erfinder: AUER, Heinz, D-68809 Neulu heim (DE); DAHLHAUS, Jürgen, B-1180 Bruxelles (BE); FISCHER, Karl, D-67591 Hohen-Sülzen (DE); HAMMER, Hans, D-68219 Mannheim (DE); KELLENBENZ, Jochen, D-67098 Bad Dürkheim (DE); SCHULZ, Michael, D-67067 Ludwigshafen (DE); THIEL, Joachim, D-67435 Neustadt (DE); VICARI, Maximilian, D-67117 Limburgerhof (DE)
(74) Vertreter: Isenbruck, Günter
(86) Internationale Anmeldenummer: PCT/EP2000/007199
(87) Internationale Veröffentlichungsnummer: WO 2001/007392

(56) Entgegenhaltungen:
- EP-A- 0 360 041
- EP-A- 0 387 482
- DE-A- 19 506 555
- DE-C- 863 046
- "Ullmann's Encyclopedia of Industrial Chemistry Vol. B3, 4-62-4-65" 1988, VCH

## Beschreibung

Die vorliegende Erfindung betrifft ein Verfahren zur Herstellung von Methylformiat, eine Vorrichtung zur Durchführung dieses Verfahrens sowie Methylformiat, das nach diesem Verfahren hergestellt wurde und das insbesondere zur Herstellung von Ameisensäure eingesetzt werden soll.

Methylformiat dient in der Technik überwiegend zur Herstellung von Ameisensäure und wird zu diesem Zweck bereits in großem Umfang durch Umsetzung von Methanol mit Kohlenmonoxid in Gegenwart von Alkalialkoholat hergestellt. Bei der technischen Umsetzung dieses Verfahrens treten jedoch Probleme auf, die das Verfahren unbequem, unzuverlässig und energiezehrend machen, und dadurch erheblich verteuern.

Mit dem Ziel, das Verfahren für den großtechnischen Einsatz zu perfektionieren, entwickelte sich die Technik zur Ausführung dieses Verfahrens im wesentlichen in zwei Richtungen: Eine Gruppe von Verfahren arbeitet unter Verwendung eines sehr hohen CO-Druckes, wobei das Ziel im Vordergrund steht, möglichst hohe Konzentrationen von Methylformiat im Reaktoraustrag zu erhalten, eine andere Gruppe von Verfahren bedient sich eines relativ geringen CO-Druckes wobei das Ziel im Vordergrund steht, durch eine relativ geringe Konzentration des Methylformiats im Reaktionsgemisch die Bildung von Salzablagerungen in den Reaktoren, Kühlflächen und Ventilen zu vermeiden, um über längere Zeit störungsfrei arbeiten zu können.

Ein Hochdruckverfahren ist aus der DE-C-926 785 bekannt, bei dem das Reaktionsgemisch im Kreislauf geführt, abwechselnd einen Reaktor und einen Kühler durchfließt. Frisches Methanol und darin gelöstes Na-methylat als Katalysator wird dem Reaktorkopf zugeführt, das Kohlenmonoxid oder das kohlenmonoxidhaltige Gasgemisch wird am Fuß des bei 80 bis 130°C arbeitenden Reaktors unter einem Druck von 300 bar eingepreßt. Ein Teil des im Kreislauf geführten Reaktionsgemisches wird kontinuierlich über eine Druckkammer ausgeschleust. Bei dem Verfahren wird mit einer geringen Katalysatorkonzentration von maximal 0,25 Gew.-% Natrium (entsprechend 0,59 Gew.-% Natriummethylat) gearbeitet, um die Salzabscheidungen möglichst gering zu halten und im Reaktor wird gerührt, um die abgeschiedenen Salzmengen in der Schwebe zu halten.

Zur Durchführung dieses bekannten Verfahrens ist ein hoher apparativer Aufwand erforderlich; insbesondere bringt das Rühren unter dem hohen Druck praktisch unlösbare technische Probleme mit sich. Trotz des hohen technischen Aufwands kann nicht verhindert werden, daß auch bei diesem Verfahren die Schwierigkeiten im Zusammenhang mit der Bildung fester Ablagerungen auftreten. Ein störungsfreier kontinuierlicher Betrieb des Verfahrens ist daher über akzeptable Zeiträume nicht möglich. Aus diesem Grund ist die in der Praxis erzielbare Produktionskapazität unbefriedigend und das Verfahren insgesamt unwirtschaftlich.

Aus der DE-C-1046602 ist ein kontinuierliches, zweistufiges Verfahren zur Herstellung von Methylformiat bekannt, bei dem in der ersten Stufe Methanol in Gegenwart von 0,5 bis 5 Gew.-% Alkalialkanolat in einem mantelgekühlten Rohrreaktor mit CO, das dem Rohreaktor in Teilströmen an mehreren verschiedenen Stellen zugeführt wird, bei einer Temperatur von 60 bis 140°C und einem Druck von 50 bis 300 bar in turbulenter Strömung so zur Reaktion gebracht wird, daß ein Methanolumsatz von 70 bis 75 % erreicht wird. Das in der ersten Stufe erhaltene Reaktionsgemisch wird dann in einem Autoklaven mit einem CO-Überschuß bis zu einem Umsetzungsgrad von ca. 90 %, bezogen auf eingesetztes Methanol, zu Methylformiat umgesetzt. Schwierig ist es bei diesem Verfahren, die im Reaktor geforderten Bedingungen, Einhaltung einer bestimmten Reaktionstemperatur bei gleichzeitig turbulenter Strömung zu gewährleisten. Dazu ist eine bei großtechnischer Durchführung des Verfahrens sehr schwierige, sehr genaue Kontrolle der Kühlwassertemperatur, des CO-Druckes und der Strömungsgeschwindigkeit der Reaktanten erforderlich. In der zweiten Stufe des Verfahrens soll die Kühlung nur noch durch Zufuhr vorgekühlten Kohlenmonoxids und/oder durch Unterkühlung der aus der ersten Stufe austretenden Reaktionsmischung erfolgen. Beide Maßnahmen erfordern einen hohen apparativen Aufwand, wenn Störungen durch Krustenbildung verhindert werden sollen. Ferner ist es erforderlich, zur Vermeidung von Niederschlagsbildung und Verschmutzungen der Kühlflächen ein besonders reines, ggf. vorgereinigtes CO einzusetzen. Obwohl sich rein rechnerisch im Ausführungsbeispiel eine Produktionskapazität von 1674 kg/m³/h ergeben sollte, konnte auch dieses Verfahren wegen des hohen technischen Aufwands, den enormen Schwierigkeiten bei der Einstellung und Überwachung der Verfahrensbedingungen und des naturgemäß verbleibenden Verstopfungs-Risikos nicht zur kontinuierlichen Produktion von Methylformiat eingeführt werden.

Die Anmelder dieses Patents haben sich intensiv bemüht, die bei diesem Verfahren auftretenden Schwierigkeiten zu überwinden. Diese Bemühungen haben zu dem in der DE-C-1 147 214 beschriebenen Verfahren geführt. Hier werden einem turmförmigen Reaktor mindestens zwei Teilströme von Kohlenmonoxid in verschiedener Höhe zugeführt, wodurch sich im Reaktor verschiedene Reaktionszonen ausbilden. In der ersten Zone wird das eingespeiste, 0,12 bis 0,3 Mol-% Alkalimethylat enthaltende Methanol unter einem CO-Druck von 150 bis 200 bar bei 30 bis 100°C zu ca. 75 bis 85 % zu Methylformiat umgesetzt, in den darunter liegenden Zonen des Reaktors erfolgt die weitere Umsetzung bei 40 bis 60°C bis zu einem Umsetzungsgrad von ca 95 %. Die bei diesem Verfahren naturgemäß ausfallenden salzartigen Niederschläge sollen durch ein in regelmäßigen oder unregelmäßigen zeitlichen Abständen vorgenommene plötzliche Veränderung der Stärke der verschiedenen CO-Teilströme, wobei die gesamt-Stromstärke konstant bleiben soll, durch Sicherstellung ausreichender Strömungs-geschwindigkeiten der Reaktionsmassen und durch wiederholtes plötzliches Öffnen und Schließen durchströmter Ventile daran gehindert werden, sich in den Anlagenteilen festzusetzen. Ausweislich des Ausführungsbeispiels erhält man bei diesem Verfahren im zeitlich begrenzten Einzelversuch allerdings nur noch eine Produktionskapazität von ca. 440 kg/m³/h.

Auch diese Verfahrensvariante erfordert einen hohen Aufwand für Überwachung und für die den Betriebswerten anzupassenden zeitlichen plötzlichen Änderungen der CO-Ströme und der Ventilsteuerung. Dennoch lassen sich bei längerer Betriebsdauer Ablagerungen des Katalysators und seiner Zersetzungsprodukte an den Anlagenteilen nicht vermeiden, was Stillstandszeiten und eine weitere Verminderung der Produktions-kapazität zur Folge hat..

Bei einem weiteren in der DE-A-195 06 555 beschriebenen Hochdruckverfahren wird Methanol in Gegenwart einer relativ geringen Katalysatorkonzentration von 0,05 bis 0,2 Gew.-% Alkalimethylat bei 50 bis 150°C mit CO unter einem Druck von 210 bis 250 bar in einem Reaktor oder einer Reaktorkaskade zur Reaktion gebracht. Durch eine besonders gute Verteilung des zugeführten Kohlenmonoxids in dem Reaktionsgemisch, die durch Einblasen desselben mittels einer Strahldüse erreicht werden soll, soll eine schnelle Umsetzung erreicht werden. Der CO-Strom kann in Teilströmen zugeführt werden und eine eventuell eingesetzte Reaktorkaskade kann ein Temperaturprofil aufweisen. Das aus dem Reaktor ausgeschleuste Reaktionsgemisch enthält ca. 97 Gew.-% Methylformiat, die Produktionskapazität liegt ausweislich der Beispiele zwischen 530 und 960 kg/m³/h.

Nicht umgesetztes Methanol wird nach der Destillation des Austrags erneut eingesetzt. Eine Rückführung des Katalysators ist jedoch nicht vorgesehen, so daß sich trotz der geringen Katalysatorkonzentration nach der Aufarbeitung des Rohprodukts relativ große Mengen an Feststoffabfällen ergeben. Darüberhinaus besteht bei Ausnutzung des im Prinzip möglichen hohen Umsetzungsgrades ein beachtliches Risiko der Abscheidung von Verkrustungen in der Anlage, so daß auch hier Stillstandszeiten der Anlage in Kauf genommen werden müssen, die die bei Einzelversuchen ermittelten an sich guten Produktionskapazitäten drastisch vermindern. Hinzu kommen die durch die hohen Verfahrensdrücke bedingten hohen Investitionskosten für die Anlage, so daß die Wirtschaftlichkeit des Verfahrens nicht voll befriedigen kann.

Die genannten Hochdruckverfahren ermöglichen einen hohen Methanol- und CO-Umsatz, führen aber zu den beschriebenen technischen Schwierigkeiten und wirtschaftlichen Nachteilen, vor allem hohen Investitionskosten und Salzab-lagerungen in den Anlagenteilen.

Eine verfahrenstechnische Alternative zu den Hochdruckverfahren sind die sogenannten Niederdruckverfahren, die bei niedrigeren CO-Drücken von ca 10 bis 100 bar arbeiten. Diese Reaktionsbedingungen führen zu einem geringeren Methanolumsatz, vermeiden allerdings in der Regel den Salzausfall. Auch Niederdruckverfahren sind in verschiedenen Ausführungsformen bereits bekannt.

Aus der Deutschen Patentschrift Nr. 863 046 ist ein Verfahren zur kontinuierlichen Herstellung von Methylformiat bekannt, zu welchem in der Deutschen Patentschrift Nr. 880 588 eine verbesserte Ausführungsform beschrieben worden ist. Bei diesem Verfahren wird eine Lösung von Natriumalkanolat mit einem Gehalt von 1 bis 2,5 Gew.-% Natrium (bei Einsatz von Methanol entsprechend 2,3 bis 5,9 Gew.-% Natriummethylat) bei 85 bis 90°C mit Kohlenmonoxid unter einem Druck von 10 bis 30 bar umgesetzt. Beim Verfahren der Deutschen Patentschrift Nr. 863 046 werden die alkanolische Natriumalkanolat-Lösung und das Kohlenmonoxid im Gegenstrom einmal durch den Reaktor geführt, beim verbesserten Verfahren der DE-C-880 588 führt man die Reaktanten im Kreislauf im Gleichstrom durch den Reaktor. Die Reaktionsbedingungen Temperatur, Strömungsgeschwindigkeit der flüssigen Phase und Druck werden so eingestellt, daß mindestens so viel des Alkanols unverändert bleibt, daß die als Katalysator verwendeten Alkalialkanolate in Lösung gehalten werden. Das bei der Passage des Reaktors entstandene Methylformiat und überschüssiges Methanol werden beim verbesserten Verfahren gemäß ihren Gleichgewichts-Dampfdrucken mit dem CO-Strom aus dem Reaktor abgeführt. Die Stärke des CO-Stromes soll so eingestellt werden, daß Methylformiat möglichst vollständig aus dem System entfernt wird. Durch möglichst weitgehende Abkühlung des aus dem Reaktor austretenden CO-Stromes werden die mitgeführten Verbindungen Methanol und Methylformiat kondensiert und die flüssige Mischung aus dem Kreislauf ausgeschleust. Das kalte Kohlenmonoxid wird danach wieder auf die Reaktionstemperatur vorgewärmt und in den Reaktor zurückgeführt.

Die so erhaltene Methanol/Methylformiat-Mischung enthält 38 bis 40 Gew.-% Methanol. Sie wird fraktioniert destilliert und das Methanol ebenfalls in den Kreislauf zurückgeführt. Bei der verbesserten Ausführungsform des Verfahrens gemäß DE-C-880 588 wird nach den Angaben im Ausführungsbeispiel aus einem Reaktor mit 770 1 Volumen pro Stunde nur eine Ausbeute von 3,1 kg Methylformiat erhalten, was einer Produktionskapazität von nur 4,0 kg/m³/h entspricht. Ein Verfahren mit einer so geringen Produktionskapazität ist für die Produktion im technischen Ausmaß völlig undiskutabel. Hinzu kommt, daß die ungewöhnlich hohe Katalysatorkonzentration erhebliche Nachteile mit sich bringt: Während der Reaktion ergibt sich nämlich generell eine fortschreitende Verminderung der Katalysatoraktivität wegen der unvermeidlichen Bildung von Alkaliformiat. Daher muß stets ein Teil des umlaufenden, katalysatorhaltigen Methanols ausgeschleust und eine entsprechende Menge frischer Katalysatorlösung zugeführt werden. Nicht unbeachtlich ist auch der für die Durchführung dieses Verfahrens erforderliche Energieverbrauch für Kühlung und Rückerwärmung des kreisenden Kohlenmonoxids und die Destillation des verdünnten Methylformiats.

Aus der Deutschen Patentschrift 22 43 811 ist ein Verfahren zur kontinuierlichen Herstellung von Methylformiat bekannt, bei dem Methanol in Gegenwart von 0,4 bis 1,5 Gew.-% Alkalimethylat bei 50 bis 130°C in einer Kolonne mit gefluteten, vorzugsweise einzeln gekühlten Böden im Gegenstrom mit Kohlenmonoxid enthaltenden Gasen umgesetzt wird.

Der CO-Partialdruck soll im Bereich von 40 bis 150 bar liegen und die Verweilzeit der Reaktanten in der Kolonne bei 50 bis 1500 Sekunden. Das nach der Kolonnenpassage im Sumpf anfallende Reaktionsgemisch enthält 20 bis 70 Gew.-% Methylformiat. Es wird destillativ aufgearbeitet.

Neben den hohen Investitionskosten für die komplizierte Kolonnenkonstruktion ist es von großem Nachteil, daß bei dem Verfahren nur ein Bruchteil der eingesetzten Komponenten Methanol und Kohlenmonoxid verbraucht werden und eine schlechte Ausnutzung des hohen Katalysatoranteils erfolgt. Dies führt zu einer weiteren hohen Kostenbelastung, die durch den Energieverbrauch für CO-Vorwärmung und destillative Aufarbeitung des gesamten, dem Kolonnensumpf entnommenen Reaktionsgemisches noch vergrößert wird. Schließlich ergeben sich noch technische Probleme bei der Beseitigung der katalysator-und salzhaltigen Destillationsrückstände.

In der US-A-4 661 624 wird ein Verfahren zur Herstellung von Methylformiat beschrieben, bei dem Methanol in Gegenwart von 1 bis 8 Mol-% (bezogen auf eingesetzten Alkohol, entsprechend 1,7 bis 13,5 Gew.-% Natriummethylat) eines Natriumalkoxid-Katalysators mit CO unter einem Druck von 5 bis 70 bar bei 70 bis 130°C umgesetzt wird. Der Prozess wird so gesteuert, daß der Umsatz auf 2 bis 10 % des eingesetzten Alkohols beschränkt ist, wodurch zwar Salzabscheidungen in den Reaktoren völlig vermieden werden, der Gehalt an Methylformiat im ausgeschleusten Reaktionsprodukt aber nur 1 bis 19 Gewichtsprozent beträgt. Das ausgeschleuste Reaktionsgemisch wird destillativ aufgearbeitet, wozu wegen des geringen Methylformiat-Gehaltes selbst bei Ausnutzung der Reaktionswärme in einem Wärmeverbund ein unvertretbar hoher Energieaufwand erforderlich ist. Das nach der Destillation erhaltene Methanol mit dem darin gelösten Katalysator wird in den Prozeß zurückgeführt. Aufgrund der hohen erforderlichen Konzentration an aktivem Katalysator ergibt sich allerdings die Notwendigkeit dem Prozeß laufend relativ große Mengen frischen Katalysators zuzuführen. Auch ist die Ausnutzung des zugeführten kohlenmonoxidhaltigen Gases nicht befriedigend.

Die DE-A-43 09 731 betrifft ein Verfahren zur Herstellung von Methylformiat, bei dem Methanol in Gegenwart von 0,4 bis 1,5 Gew.-% Alkalimetallmethylat bei 60 bis 100°C unter einem Druck von 10 bis 300 bar (im Ausfiilirungsbeispiel 57 bar) mit Kohlenmonoxid oder kohlenmonoxidhaltigen Gasen in einer Mischzone teilweise umgesetzt wird. Das dort erhaltene Gemisch wird mit CO gesättigt und einer Nachreaktionszone zugeführt, wo die Umsetzung ohne Zufuhr weiterer Ausgangsmaterialien zu Ende geführt wird. Auch bei diesem Verfahren wird nur eine geringe Konzentration des Methylformiats im Reaktionsaustrag erhalten , so daß sich eine ungünstige Energiebilanz für den Prozeß ergibt.

Aus der DE-C-27 10 726 ist ein Verfahren zur Herstellung von Methylformiat bekannt, bei dem eine Lösung von 0,2 bis 2,5 Gew.-% eines Alkali- oder Erdalkalimethylat in Methanol im Kreislauf durch einen Reaktor geführt wird, der in einem unteren Abschnitt die flüssige Phase, in seinem oberen Teil eine 20 bis 100 Vol.-% Kohlenmonoxid enthaltende Gasphase enthält. Die Reaktion erfolgt bei 70 bis 110°C und einem Druck von 20 bis 110 bar. Die methanolische Katalysatorlösung wird unter Benutzung einer Vorrichtung (z.B. Prallplatte), die den mit hoher Geschwindigkeit eingespritzten Flüssigkeitsstrahl in der Gasphase zerstäubt, oder die Gas aus der Gasphase ansaugt und feinblasig in die Flüssigphase injiziert (z.B. Venturidüse), dem Reaktor zugeführt.

Im Flüssigkeitskreislauf liegt ein Wärmetauscher, der eine genaue Temperatureinstellung der kreisenden Flüssigkeit gestattet und eine starke Pumpe, die den turbulenten Flüssigkeitskreislauf aufrechterhält. Im Betrieb wird dem Reaktor kontinuierlich frische methanolische Methylatlösung zugeführt und eine entsprechende Menge des Reaktionsgemisches ausgeschleust, das destillativ aufgearbeitet werden muß.

Nach den Angaben dieser Druckschrift ist es aus thermodynamischen Gründen zweckmäßig, die Zufuhr von Ausgangsmaterialien und die Ausschleusung so einzustellen, daß das Reaktionsgemisch ca. 44 bis 65 Gew.-% Methylformiat enthält. Nimmt man die Bildung von Ausfällungen in Kauf, so kann ein Reaktoraustrag mit einem Methylformiatgehalt von 82 Gew.-% erhalten werden. Solche Ausfällungen sollen zwar nach Angaben der Druckschrift tolerierbar sein, dies gilt jedoch nur für einen zeitlich begrenzten Einzelversuch. Auf jeden Fall ergibt sich durch Ausfällungen eine erhöhte Abrasion der hoch belasteten Umlaufpumpe und der ggf. eingesetzten Prallelemente bzw. der Venturidüse. Die Isolierung des Methylformiats aus einer Mischung, die nur 44 bis 65 Gew.-% dieser Substanz enthält, führt zu hohen Energiekosten. Hohe Betriebskosten des Verfahrens ergeben sich auch durch den sich aus der hohen Katalysatorkonzentration (in 5 von 6 Ausführungsbeispielen 2,5 Gew.-%) ergebenden hohen Katalysatorverbrauch und durch den Aufwand für die sachgemäße Entsorgung des Destillationsrückstands. Ein weiterer Nachteil des Verfahrens besteht darin, daß bei einer Erhöhung des Methylformiat-Gehalts im Austrag eine Verringerung der Produktionskapazität eintritt.

Es sind auch verschiedene Versuche gemacht worden, die mit den oben beschriebenen bekannten Verfahren verbundenen Nachteile und Schwierigkeiten auf anderen als den bisher beschrittenen Wegen zu überwinden.

So ist es beschrieben worden, durch Zusätze von Komplexbildnern insbesondere von cyclischen Polyethern (EP-B-0 048 891), von oberflächenaktiven Lösungsvermittlern wie Alkali- perfluoralkansulfonaten (EP-B-0 596 483) oder von inerten, polaren, aprotischen Lösungsmitteln (EP-A-0 251 112) die Bildung von Niederschlägen zu verhindern und/oder die Produktionskapazität zu verbessern. Andere Versuche beinhalten den Einsatz anderer Katalysatoren wie z.B. von Amidin-Derivaten (EP-A-0 104 875) oder von Kombinationen von Aminen mit Ethylenoxid. Diese Verfahren haben den gemeinsamen Nachteil, daß sie zusätzliche organische Materie in die Reaktionsgemische einführen, die nach der Aufarbeitung entsorgt werden müssen, was die durch eventuell erzielte Ausbeuteverbesserungen erhaltenen Vorteile zunichte macht. Außerdem stellt der Einstandspreis für die vorgeschlagenen Zusätze ein unüberwindliches Hindernis für ihren großtechnischen Einsatz dar.

Es wurde nun überraschenderweise gefunden, daß man ohne komplizierte Zusätze Methylformiat störungsfrei, mit guten Ausbeuten und überaus kostengünstig herstellen kann, wenn man nach dem im Folgenden beschriebenen Verfahren arbeitet.

Ein Gegenstand der vorliegenden Erfindung ist ein Verfahren zur Herstellung von Methylformiat durch Umsetzung von überschüssigem Methanol mit Kohlenmonoxid unter erhöhtem Druck und erhöhter Temperatur in Gegenwart von Alkali- oder Erdalkalimethylat als Katalysator in einem druckfesten Reaktor, Abtrennung des gebildeten Methylformiats aus dem Reaktoraustrag und Rückführung der im wesentlichen methylfonniatfreien Flüssigphase in den Reaktor, wobei ein Teil der rückzuführenden Flüssigphase ausgeschleust und frische Katalysatorlösung zugeführt wird, das dadurch gekennzeichnet ist, daß
die Umsetzung in einer Kaskade aus mindestens 2 Reaktorelementen
bei einer Temperatur von 80 bis 120°C,
unter einem Kohlenmonoxid-Druck von 90 bis 180 bar,
in Gegenwart von 0,05 bis 0,5 Gew.-%, bezogen auf das Gewicht des flüssigen Feeds, eines Alkali- oder Erdalkalialkoholats ausgeführt wird,
daß das Verhältnis der in der Zeiteinheit zugeführten Mengen der Ausgangsmaterialien, die Reaktionstemperatur, der Druck und die Verweilzeit der Reaktanten in den Reaktorelementen so eingestellt werden, daß mindestens soviel des Methanols unumgesetzt bleibt, daß sowohl der verwendete Katalysator als auch , dessen Abbauprodukte unter den Reaktionsbedingungen im Reaktor und im frischen Reaktoraustrag praktisch vollständig gelöst bleiben,
daß der gesamte Reaktoraustrag einer Stripvorrichtung zugeführt wird, in der im wesentlichen das Methylformiat aus der Reaktionsmischung ausgetrieben wird,
daß ein Teil TR der verbleibenden Flüssigphase in den Reaktor zurückgeführt und, in Abhängigkeit vom Alkali- oder Erdalkaliformiat-Gehalt des entgasten Reaktoraustrags, ein Teil TA von 20 bis 80 % der verbleibenden Flüssigphase ausgeschleust wird, wodurch erreicht wird, daß an keiner Stelle des Verfahrens feste Abscheidungen von Alkali- oder Erdalkalisalzen auftreten,
daß dem ausgeschleusten Teil in einer Entsalzungsvorrichtung Restkatalysator und Katalysator-Abbauprodukte feststofffrei entzogen werden, und das verbleibende Methanol unmittelbar oder mittelbar in den Reaktor zurückgeführt wird.

Zweckmäßigerweise werden zur Durchführung des erfindungsgemäßen Verfahrens 2 bis 5, vorzugsweise 2 bis 4, kaskadierte Reaktorelemente eingesetzt. Die flüssige Phase des Reaktionsgemisches, bestehend im Wesentlichen aus Methanol, Methylformiat und gelöstem aktivem und verbrauchtem Katalysator, kann im Gleich- oder Gegenstrom zur gasfönnigen Phase, bestehend im wesentlichen aus CO bzw. dem CO enthaltenden Gasgemisch, durch die Reaktorkaskade geführt werden. Bevorzugt ist der Gegenstrombetrieb.

Bevorzugt zur Durchführung des erfindungsgemäßen Verfahrens ist der Temperaturbereich von 90 bis 110°C. Der bevorzugte Druckbereich erstreckt sich von 110 bis 160, insbesondere von 110 bis 140 bar. Es ist selbstverständlich, daß man auch bei höheren Drücken arbeiten könnte, wodurch jedoch einer der Vorteile des erfindungsgemäßen Verfahrens verloren ginge.

Der beim erfindungsgemäßen Verfahren eingesetzte Katalysator ist ein Alkali- oder Erdalkalialkoholat, vorzugsweise ein Methylat. Bevorzugt sind Alkalimetallmethylate insbesondere Natrium- und Kaliummethylat, die auch im Gemisch miteinander eingesetzt werden können. Unter besonderen Verfahrensbedingungen, z.B. beim Auftreten höherer als der üblichen Methylformiatkonzentrationen in einzelnen Verfahrensstufen, kann der Einsatz von Kaliummethylat Vorteile bringen. Auch rein ökonomische Gesichtspunkte z.B. der Gestehungspreis und/oder die Verfügbarkeit der Katalysatorsubstanzen, können ggf. die Entscheidung für einen der möglichen Katalysatoren oder für den Einsatz einer Kombination derselben beeinflussen.

Der für die Reaktion eingesetzte Katalysator wird durch unvermeidliche Nebenreaktionen zu katalytisch unwirksamen Substanzen umgesetzt. So wird durch Spuren von Wasser Alkali- bzw. Erdalkalihydroxid gebildet, Spuren von CO₂ führen zur Bildung von Karbonaten, die Reaktion des Alkoholats mit dem hergestellten Methylformiat führt zu Alkali- bzw. Erdalkaliformiat und auch durch direkte Reaktion von Alkali- und Erdalkalihydroxiden mit Kohlenmonoxid entstehen als Nebenprodukt Alkali- bzw. Erdalkaliformiate.

Alle aus dem Katalysator durch Nebenreaktionen gebildeten, katalytisch inaktiven Produkte werden im Folgenden gemeinsam als "verbrauchter Katalysator" oder als "Katalysator-Abbauprodukte" bezeichnet.

Die bevorzugte Katalysatorkonzentration beträgt 0,1 bis 0,3 Gew.-%, beispielsweise 0,2 Gew.-%, bezogen auf das Gewicht des flüssigen Feeds.

Es hat sich überraschenderweise gezeigt, daß durch die Kaskadierung der Reaktorelemente der Verbrauch des Katalysators durch Nebenreaktionen deutlich herabgesetzt werden kann. Dies könnte darauf zurückzuführen sein, daß durch die Kaskadierung der Reaktorelemente die Rückmischung der in den einzelnen Reaktorelementen enthaltenen Reaktionsgemische weitgehend unterbunden wird, was die weitere vorteilhafte Konsequenz hat - der bei herkömmlichen Verfahren anscheinend keine Beachtung geschenkt wurde - daß beim erfindungsgemäßen Verfahren, im Gegensatz zu vielen herkömmlichen Verfahren, im Bereich der Katalysatorzuführung eine vergleichsweise niedrige Methylformiat-Konzentration herrscht.

Hieraus ergibt sich der Vorteil, daß trotz geringer Katalysatorkonzentrationen hohe, ökonomisch wertvolle Umsätze erzielt werden können und daß nur relativ geringe Mengen an verbrauchtem Katalysator aus dem Verfahren ausgeschleust werden müssen.

Bei den oben angegebenen Reaktionsbedingungen werden in geradem Durchgang der Reaktanten durch den Reaktor und die angeschlossenen Aufarbeitungselemente ca. 30 % des eingesetzten Katalysators verbraucht. Bei der Beurteilung dieses Prozentsatzes ist zu berücksichtigen, daß für die Wirtschaftlichkeit des Verfahrens der Absolutwert des Katalysatorverbrauchs ausschlaggebend ist. Dieser Absolutwert ist wegen der geringen erfindungsgemäß erforderlichen Katalysatorkonzentration in der Synthese erheblich geringer als bei bekannten Verfahren. Natürlich ist es erforderlich, eine der über die Entsalzungsstufe ausgeschleusten Menge unverbrauchten und verbrauchten Katalysators entsprechende Menge einer methanolischen Lösung frischen Katalysators in den Reaktor einzuschleusen.

Der zu ergänzende Katalysator wird in Form einer 10 bis 50 gew.-%igen (in diesem Bereich möglichst konzentrierten), vorzugsweise 20 bis 40 gew.-%igen, beispielsweise 30 gew.-%igen methanolischen Lösung zugeführt.

Zweckmäßigerweise wird die Zufuhr der Reaktanden zum Synthesereaktor so geregelt, daß das Molverhältnis von Methanol zu Kohlenmonoxid 3:1 bis 0,5:1, vorzugsweise 2:1 bis 1:1 1 beträgt.

Ferner erhöht es die Variabilität des Verfahrens, wenn die Gasaufgabe wahlweise am ersten oder an den beiden ersten Reaktorelementen der Reaktorkette erfolgt, wobei die Mengen der den einzelnen Elementen zugeführten Gasmengen nach Bedarf variiert werden können, um darin bestimmte erwünschte Reaktionsbedingungen herzustellen.

Die Zählung der Reaktorelemente beginnt an demjenigen Ende der Reaktorkette an dem das Kohlenmonoxid eingeleitet (Figur, Nr. 5) und der Reaktoraustrag abgenommen wird (Figur, Nr. 6).

Für die Durchführung des erfindungsgemäßen Verfahrens hat es sich als besonders zweckmäßig erwiesen, die Reaktionsbedingungen in den Reaktorelementen so einzustellen, daß in einem ersten von zwei Teilabschnitten des Reaktionsweges 75 bis 95 %, vorzugsweise 80 bis 95 %, der Reaktionswärme, in dem zweiten Teilabschnitt 5 bis 25 %, vorzugsweise 5 bis 15 %, der Reaktionswärme auftreten. Hierbei können die Teilabschnitte, insbesondere der erste Teilabschnitt, auf mehrere Reaktorelemente verteilt sein. Bevorzugt ist es, die Reaktion in drei Reaktorelementen auszuführen und so zu steuern, daß im ersten und zweiten Element jeweils 35 bis 50 %, im dritten Element 5 bis 15 % der Reaktionswärme auftreten. Beispielsweise können im ersten, zweiten und dritten Reaktorelement 50%, 40% und 10% oder 46%, 46% und 8% der Reaktionswärme freigesetzt werden.

Es ist auch möglich die Reaktorkaskade so zu betreiben, daß das letzte Reaktorelement in der Kette nur durch die Reaktanden-Zufuhr gekühlt wird.

Für die Ökonomie des erfindungsgemäßen Verfahrens ist es besonders vorteilhaft, die Reaktionsbedingungen so zu wählen, daß der Reaktoraustrag 5 bis 40, vorzugsweise 10 bis 25 Gew.-% Methanol und 60 bis 95, vorzugsweise 75 bis 90 Gew.-% Methylformiat neben verbrauchtem und unverbrauchtem Katalysator enthält.

Ferner ist es äußerst zweckmäßig die Reaktandenzufuhr und die Reaktionsbedingungen so aufeinander abzustimmen, daß 90 bis 99 %, vorzugsweise 95 bis 99 % des zugeführten Kohlenmonoxids zu Methylformiat umgesetzt wird.

Kohlenmonoxid, Methanol und Katalysator werden in der Reaktionszone vermischt, wobei eine gute Dispergierung des Gases eine schnelle Umsetzung ermöglicht. Beispielsweise ist es vorteilhaft, das Gas durch eine Strahldüse in den Reaktor einzuführen oder durch einen geeigneten Rührer z.B. einen Schnellrührer, in dem Reaktor zu dispergieren. Auch eine Durchmischung durch einen äußeren Kreislauf für jedes Reaktorelement ist vorteilhaft. Der Kohlenmonoxidstrom kann an einer Stelle bzw. in ein Reaktorelement, oder aufgeteilt in mehrere Teilströme an verschiedenen Stellen des Reaktors bzw. in verschiedene Reaktorelemente eingespeist werden.

An die Synthese des Methylformiats schließt sich beim erfindungsgemäßen Verfahren die feststofffreie Aufarbeitung des in der Reaktorkaskade erhaltenen Reaktionsgemisches an. Prinzipiell kann die Aufarbeitung unter dem bei der Synthese angewendeten Druck erfolgen. Viel günstiger - insbesondere im Hinblick auf die für die destillative Aufarbeitung benötigten Temperaturen - ist es jedoch, den Reaktoraustrag vor der Strip-Vorrichtung auf einen Druck von 5 bis 25 bar, vorzugsweise von 10 bis 20 bar, zu entspannen. Um die Kreislaufströme so gering wie möglich zu halten und um eine Kondensation im Kompressor zu vermeiden, wird das hierbei in relativ geringer Menge erhaltene Sprudelgas zur Abscheidung mitgeführten Methanols und Methylformiats abgekühlt, vorzugsweise etwa auf Raum- oder Umgebungstemperatur, dann zweckmäßigerweise komplett vor den CO-Kompressor zurückgeführt und erneut in die Synthese eingespeist.

Aus der gesamten, nach der Entspannung verbleibenden Flüssigphase des Reaktoraustrags, die beispielsweise aus einem Gemisch mit etwa 75 Gew.-% Methylformiat, knapp 25 Gew.-% Methanol und einem Rest an Katalysator (zerstört und unzerstört) sowie Spuren von Dimethylether, Kohlenoxid und weiteren Inerten besteht, wird anschließend in einer Destillationsvorrichtung die Hauptmenge des Methylformiats verdampft. Gleichzeitig wird naturgemäß auch ein gewisser Anteil des im Reaktoraustrag enthaltenen Methanols - verdampft. Für das Abdampfen des Methylformiats wird zweckmäßigerweise der Druck über dem Reaktoraustrag nochmals soweit abgesenkt daß sich der Kopfdruck der Destillationsvorrichtung auf 1,2 bis 2,5 bar, vorzugsweise auf 1,5 bis 2,0 bar, einstellt. Die für das Abdampfen erforderliche Verdampfungswärme ist in der Regel zumindest zum Teil bereits in dem heißen Reaktoraustrag enthalten. Der Rest wird durch direkte Beheizung der Destillationsvorrichtung oder durch Zufuhr von Wärmeträgern gedeckt.

Je nach dem gewünschten Reinheitsgrad des hergestellten Methylformiats wird das Rückflußverhältnis in der Destillationsvorrichtung so eingestellt, daß über Kopf 85 bis 90 gew.-%iges Methylformiat abgezogen werden kann.

Der nach der Abtrennung des Methylformiats verbleibende Teil des Reaktoraustrags besteht im wesentlichen aus aktiven und verbrauchten Katalysator enthaltendem Methanol. Diese Lösung wird in den Teil TR, der ohne weiteres in die Reaktorkaskade zurückgeführt wird, und in den Teil TA, der aus dem Kreislauf ausgeschleust wird, geteilt. Wie oben bereits ausgeführt, wird das Teilungsverhältnis TR:TA in Abhängigkeit vom Alkali- oder Erdalkaliformiat-Gehalt des entgasten Reaktoraustrags gesteuert, wobei es das Ziel ist, das Teilungsverhältnis so festzulegen, daß an keiner Stelle des Verfahrens feste Abscheidungen von Alkali- oder Erdalkalisalzen auftreten. Erreicht wird dieses Ziel, indem man TR und TA so einstellt, daß eine solche Menge desaktivierter Katalysator über die Entsalzungsvorrichtung den Prozeß verläßt, wie beim geraden Durchgang durch den Reaktor und in den weiteren Verfahrensstufen verbraucht wird. Dadurch gelangt selbstverständlich auch noch aktiver Katalysator in die Entsalzungsvorrichtung, so daß dieser Anteil den Prozeß verläßt.

Es ist vorteilhaft das Teilungsverhältnis so zu steuern, daß der Alkali- oder Erdalkaliformiat-Gehalt am Reaktoraustritt 0,05 bis 0,5 , vorzugsweise 0,1 bis 0,3 Gew.-%, beträgt.

Erfahrungsgemäß werden zur Aufrechterhaltung eines konstanten Synthesebetriebs 20 bis 80 % des nach der Abtrennung des Methylformiats verbleibenden katalysatorhaltigen Methanols ausgeschleust.

Der ausgeschleuste Anteil TA des Alkali- und/oder Erdalkalialkoholate als Katalysator und Katalysator-Abbauprodukte enthaltenden Methanols wird anschließend feststofffrei entsalzt. Hierzu wird dieser Flüssigphase in der Entsalzungsvorrichtung soviel Wasserdampf und/oder Heißwasser und gegebenenfalls zusätzliche Wärme zugeführt, daß das Methanol im wesentlichen vollständig verdampft, und eine wäßrige Lösung der Katalysatorabbauprodukte erhalten wird. Als "Katalysatorabbauprodukt" wird hier auch das aus aktivem Restkatalysator und Wasser gebildete Alkali- oder Erdalkalihydroxid bezeichnet.

Bei dieser Operation wird die Menge an Wasserdampf und/oder Heißwasser so bemessen, daß der Katalysator und seine Abbauprodukte sicher in wäßriger Lösung gehalten werden. In der Regel ist diese Bedingung erfüllt, wenn die Konzentration der Katalysatorabbauprodukte im wäßrigen Austrag der Entsalzungsvorrichtung 5 bis 20 Gew.-%, vorzugsweise 6 bis 12 Gew.-%, beispielsweise 8 Gew.-% beträgt.

Vorzugsweise wird der Entsalzungsvorrichtung Wasserdampf mit einem Druck von 2 bis 6 bar, vorzugsweise 3 bis 5 bar, z.B. in Form von Direktdampf, zugeführt.

Von besonderem ökonomischen Interesse ist es, die Entsalzungsvorrichtung mit der Destillationsvorrichtung in einem Wärmeverbund zu betreiben, wobei der über Kopf der Entsalzungsvorrichtung entweichende Methanoldampf der Destillationsvorrichtung zugeführt wird.

Besondere Vorteile ergeben sich in diesem Fall, wenn der Kopfdruck der Entsalzungsvorrichtung so gewählt wird, daß ihr Brüdenstrom aus ihrem Kopf direkt in die Destillationsvorrichtung eingespeist werden kann.

Das hier benutzte Verfahren, aktiven Restkatalysator und verbrauchten Katalysator enthaltendes Methanol feststoffrei zu entsalzen ist ebenfalls ein Gegenstand der vorliegenden Erfindung. Der besondere Wert dieses Verfahrensteils besteht darin, daß dadurch die Möglichkeit eröffnet wird, die gesamte Methylformiat-Synthese feststofffrei auszuführen.

Außer dem Katalysator ist der Reaktorkaskade auch das bei der Synthese verbrauchte Methanol zuzuführen. Das bei dem erfindungsgemäßen Verfahren eingesetzte Methanol ist chemisch oder technisch reines Methanol. Es kann auch aus anderen Prozessen, z.B. der Hydrolyse von Methylformiat zu Ameisensäure und Methanol, rückgeführt werden. Um einen möglichst geringen Katalysatorverbrauch zu gewährleisten wird vorzugsweise Methanol eingesetzt, dessen Wassergehalt unter 100 ppm, vorzugsweise unter 30 ppm, insbesondere im Bereich von 5 bis 15 ppm liegt.

Aus dem gleichen Grund und um einen möglichst hohen Umsatz der Reaktanden zu erzielen ist es zweckmäßig, Kohlenmonoxid in Form eines kohlenmonoxidhaltigen Gasgemisches einzusetzen mit einem CO-Gehalt von 45 bis 100 Vol.-%, vorzugsweise 70 bis 100 Vol.-%, insbesondere 90 bis 100 Vol.-%. Beim Einsatz derartiger Gasgemische wird der Gesamtdruck vorzugsweise so eingestellt, daß der Kohlenmonoxid-Partialdruck im Bereich von 90 bis 180 bar liegt.

Die CO enthaltende Gasphase kann Gase, die unter den Synthesebedingungen inert sind, wie z.B. Wasserstoff, Stickstoff, Kohlenwasserstoffe, enthalten.

Unter bestimmten Bedingungen, z.B. wenn das erfindungsgemäße Verfahren mit anderen Verfahren im Verbund betrieben wird oder besonders günstige Beschaffungskonditionen vorliegen, kann es auch ökonomisch vorteilhaft sein, das erfindungsgemäße Verfahren mit Abgas mit einem Gehalt von ca. 50 Vol.-% CO zu betreiben. Aufgrund der hohen Anpassungsfähigkeit des Verfahrens an Änderungen der Verfahrensbedingungen ist auch unter Einsatz eines solchen Abgases eine gute Ausbeute von Methylformiat in jeder gewünschten Reinheit zu erzielen. Üblicherweise werden mit dem erfindungsgemäßen Verfahren Ausbeuten an Methylformiat von über 90 Mol.-% und sogar von über 95 Mol.% (bezogen auf eingesetztes CO) erreicht. Bezogen auf eingesetztes Methanol liegen die Ausbeuten - bei Einsatz von hochprozentigem oder reinem CO - praktisch bei 100 Mol.%.

Ein weiterer Vorteil des erfindungsgemäßen Verfahrens besteht darin, daß das aus der Entsalzungsvorrichtung ausgeschleuste Abwasser nur 15 bis 30 ppm, in der Regel ca. 20 ppm, d.h. praktisch kein Methanol enthält.

Der Betrieb der im Verfahren benutzten Kühler erfolgt mit Fluß- und/oder Kaltwasser.

Durch das erfindungsgemäße Verfahren kann bei niedrigen Katalysatorkonzentrationen, und bei mittleren Drücken, eine hohe Raum-Zeit-Ausbeute von 400 bis über 1000 kg/m³/h, bei Methylformiat-Endkonzentrationen von über 80 Gew.-% erzielt werden. Die niedrige erforderliche Katalysatorkonzentration trägt dazu bei, die Einsatzstoff-Kosten deutlich zu senken, durch den moderaten Synthesedruck werden die hohen Investitionskosten für Hochdruckanlagen vermieden. Weiterhin erlaubt das erfindungsgemäße Verfahren die Anlage so zu steuern, daß keine Salzausfällungen auftreten; dadurch entfallen Verstopfungsprobleme, die Verfügbarkeit der Anlage und damit die Jahreskapazität wird erheblich gesteigert. Der Methanolumsatz ist wesentlich höher als bei den typischen Niederdruckverfahren und somit ist auch die im Kreis zu führende Methanolmenge erheblich geringer. Die erfindungsgemäße Aufarbeitung erfordert sehr wenig Energie und führt trotzdem zu einem Methylformiat, dessen Konzentration so hoch ist, daß es direkt für weitere Reaktionen, wie z.B. der Hydrolyse zu Ameisensäure eingesetzt werden kann.

Ein weiteres vorteilhaftes Merkmal des erfindungsgemäßen Verfahrens ist seine große Anpassungsfähigkeit an Änderungen der Reaktionsbedingungen, die man zur Einstellung bestimmter, gewünschter Qualitäten des Endprodukts vornehmen will. Ferner kann das Verfahren auch ohne Nachteile in ein und der selben Apparatur je nach Verfügbarkeit auch mit verschiedenen Katalysatoren, Katalysatormengen und unterschiedlichen CO-Qualitäten betrieben werden. Technisch besonders vorteilhaft ist auch die Tatsache, daß man die Parameter des erfindungsgemäßen Verfahrens in weiten Grenzen variieren kann, ohne daß es zu Ausfällungen von Katalysator und Katalysatorabbauprodukten, wie z.B. Alkali- oder Erdalkaliformiaten, kommt. Es treten daher keine festen Ablagerungen auf den inneren Oberflächen der Anlage, insbesondere an den Wärmetauscherflächen, auf, so daß Probleme mit der Temperaturführung in den einzelnen Anlagenteilen und mit Rohrverstopfungen, wie sie bei bekannten Hochdruckverfahren regelmäßig auftreten, völlig vermieden werden.

Der Umsatz der Reaktion läßt sich sowohl über die Lage des thermodynamischen Gleichgewichts - beeinflußbar durch die Einstellungen von Druck und Temperatur - als auch über die Stöchiometrie und/oder die Verweilzeit der Reaktanten im Reaktor steuern. Hohe Endumsätze verringern die Mengen der in die Reaktion zurückzuführenden, bei der Aufarbeitung anfallenden unverbrauchten Ausgangsstoffe. Bewährt haben sich CO-Umsätze von 90 bis 99 % der Theorie.

Ein weiterer Vorteil des erfindungsgemäßen Verfahrens besteht darin, daß aufgrund des hohen CO-Umsatzes, im Gegensatz zu vielen bekannten Verfahren entweder überhaupt keine Rückführung von nicht umgesetztem CO erforderlich ist, - wodurch die Investitionskosten für Kompressoren wegfallen - oder, falls eine Rückführung der geringen nicht umgesetzten Menge von Kohlenmonoxid gewünscht wird, dies mit einem Minimum an Kompressionsarbeit bewerkstelligt werden kann.

Das erfindungsgemäße Verfahren mit seinem erheblich über dem gängiger Niederdruckverfahren liegenden Arbeitsdruck von 90 bis 160 bar ist nach gängiger Nomenklatur ein Mittel- bis Hochdruckverfahren. Bei bekannten, im Bereich über 100 bar arbeitenden Verfahren wird kein Katalysator zurückgeführt. Der gesamte eingesetzte Katalysator ist nach einmaliger Reaktorpassage verloren. Eine weitere bei bekannten Hoch- und Mitteldruckverfahren auftretende Komplikation besteht in der Bildung von Methylformiat/Methanol/Salz-Suspensionen, aus denen die Salze nur durch ein aufwendiges Feststoffhandling abgetrennt werden können.

Bei bekannten Nieder- und Mitteldruckverfahren, bei denen eine Katalysatorrückführung erforderlich ist, wird die Einführung von Wasser in den Stoffkreislauf strikt vermieden, weil der Katalysator durch Wasser zerstört wird. Da auch bei diesen Verfahren verbrauchter Katalysator aus dem System entfernt werden muß, muß er in diesen Fällen ebenfalls durch Methoden der Feststoff-Verfahrenstechnik, z.B. durch Abfiltrieren oder Abzentrifugieren, ausgeschleust werden.

Überraschend wurde nun gefunden, daß beim erfindungsgemäßen Verfahren ein Teil des nicht verbrauchten Katalysators zurückgeführt werden kann, ohne daß Verkrustungen in den Apparateteilen, insbesondere an den Wärmetauschern, auftreten. Außerdem wird beim erfindungsgemäßen Verfahren das technisch aufwendige Arbeiten mit Feststoffen vollständig vermieden, da es überraschenderweise gelungen ist, diese Trennung auf rein thermisch-destillativem Weg feststofffrei durchzuführen und das Verfahren so zu gestalten, daß verbrauchter Katalysator als wässrige Lösung ausgeschleust, und trotz der Wasserzufuhr ein beachtlicher Teil des unverbrauchten Katalysators in die Synthese zurückgeführt werden kann.

Ein weiterer Gegenstand der vorliegenden Erfindung ist eine Anlage zur Produktion von Methylformiat nach dem oben beschriebenen erfindungsgemäßen Verfahren, mit
A) einer Synthesegruppe im wesentlichen bestehend aus
   A1) einem Reaktor mit mindestens zwei separat heiz- und kühlbaren Reaktorelementen mit Zuführungen für frisches Methanol, rückgeführtes katalysatorhaltiges Methanol, für frische methanolische Katalysatorlösung und für ein Kohlenmonoxid enthaltendes Gasgemisch, mindestens je einer Ableitung für den Reaktoraustrag und Restgas, Vorrichtungen zur Erzeugung und Aufrechterhaltung einer Feinverteilung des Gasstromes in dem Flüssigkeitsstrom Meßvorrichtungen zur Temperatur- und Druckkontrolle,
   A2) einer mit Kühlelementen versehenen Entspannungsvorrichtung zur Entspannung des Reaktoraustrags auf den Aufarbeitungsdruck mit einer Zuführung für den Reaktoraustrag und Ableitungen für Restgas und Flüssigphase
B) einer Aufarbeitungsgruppe im wesentlichen bestehend aus
   B1) einer Destillationsvorrichtung zur Abtrennung von im wesentlichen Methylformiat aus der Flüssigphase des Reaktoraustrags, mit einer Zuführung für die Flüssigphase und Ableitungen für im wesentlichen Methylformiat und für verbleibendes katalysatorhaltiges Methanol,
   B2) einem regelbaren Stromteiler für die Aufteilung des die Destillationsvorrichtung verlassenden, Restkatalysator und Katalysatorabbauprodukte enthaltenden Methanol-Stromes in die Anteile TR und TA,
   B3) einer gegebenenfalls mit Heiz- und Kühlelementen versehenen, feststofffrei arbeitenden Entsalzungsvorrichtung, mit Einlässen für Restkatalysator und Katalysatorabbauprodukte enthaltendes Methanol und Heißwasser oder Wasserdampf und Auslässen für Methanoldampf und wäßrige Salzlösung,
C) Verbindungsleitungen und erforderlichenfalls Pumpen für eine zweckentsprechende Förderung der Reaktionsteilnehmer und -produkte zwischen den Elementen der Anlagenteile A und B und Zuführungen für Ausgangsmaterialien und Ableitungen für Methylformiat und Abgas.

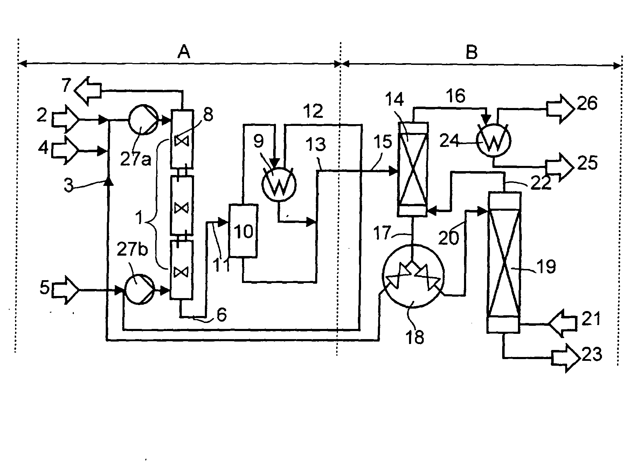

Die Figur veranschaulicht beispielhaft und schematisch die erfindungsgemäße Anlage zur Herstellung von Methylformiat und die darin integrierte erfindungsgemäße Entsalzungsvorrichtung, die gemäß einer bevorzugten Ausführungsform mit der Destillationsvorrichtung im direkten Wärmeverbund arbeitet.

Sie zeigt im Abschnitt A die Synthesegruppe bestehend aus einem Reaktor (1) mit drei Reaktorelementen mit Zuführungen für frisches Methanol (2), rückgeführtes katalysatorhaltiges Methanol (3), für frische methanolische Katalysatorlösung (4) und für ein Kohlenmonoxid enthaltendes Gasgemisch (5), je einer Ableitung für den Reaktoraustrag (6) und Restgas (7), Vorrichtungen zur Erzeugung und Aufrechterhaltung einer Feinverteilung des Gasstromes in dem Flüssigkeitsstrom (8) einer mit Kühlelementen (9) versehenen Entspannungsvorrichtung (10) zur Entspannung des Reaktoraustrags auf den Aufarbeitungsdruck, mit einer Zuführung (11) für den Reaktoraustrag und Ableitungen für Restgas (12) und Flüssigphase (13).

Im Abschnit B zeigt die Figur die Aufarbeitungsgruppe bestehend aus einer Destillationsvorrichtung (14) zur Abtrennung von im Wesentlichen Methylformiat aus der Flüssigphase des Reaktoraustrags, mit einer Zuführung (15) für die Flüssigphase und Ableitungen für im Wesentlichen Methylformiat (16) und für verbleibendes katalysatorhaltiges Methanol (17), einem regelbaren Stromteiler (18) für die Aufteilung des die Destillationsvorrichtung verlassenden, Restkatalysator und Katalysatorabbauprodukte enthaltenden Methanol-Stromes in die Anteile TR und TA, der feststofffrei arbeitenden Entsalzungsvorrichtung (19) mit Einlässen für Restkatalysator und Katalysatorabbauprodukte enthaltendes Methanol (20) und Heißwasser oder Wasserdampf (21) und Auslässen für Methanoldampf (22) und wäßrige Salzlösung (23), einer Kühlvorrichtung (24) für das von der Ableitung (16) der Destillationsvorrichtung (14) kommende Methylformiat und Auslässen für das gekühlte Methylformiat (25) und für Restgas (26). Ferner zeigt die Figur die Pumpen (27a und 27b) als Hilfsaggregate.

Die Reaktorelemente können die Bauformen von Kessel- oder Rohrreaktoren haben. Es ist nicht erforderlich, jedes Reaktorelement als Einzelaggregat auszubilden. Vielmehr kann jede konstruktive Ausgestaltung eines Reaktors eingesetzt werden, die die Funktion einer Serienschaltung mehrerer Reaktorelemente erfüllt. Es ist daher auch ohne weiteres möglich, einen Einzelreaktor einzusetzen der durch geeignete Einbauten in mindestens zwei, vorzugsweise 2 bis 5, insbesondere 2 bis 4, Reaktionszonen, unterteilt ist. Wenn im Folgenden von Reaktorelementen gesprochen wird, soll dieser Begriff auch die in einem Einzelreaktor abgeteilten Reaktionszonen einschließen. Umgekehrt schließt auch der Begriff "Reaktionszone" einzelne Reaktorelemente ein. Auch ein solcher Reaktor kann die Bauformen von Kessel- oder Rohrreaktoren haben.

Die Reaktorelemente weisen zweckmäßigerweise eigene, regelbare Zu- und Abführungen für Reaktanden und Austräge und gegebenenfalls eigene heiz- und/oder kühlbaren Außenkreisläufe auf. Ferner können Temperatur und Druck der Reaktorelemente einzeln gesteuert werden. Die Kaskade kann ein Temperaturprofil aufweisen oder aber bei einer einheitlichen Temperatur betrieben werden. Reaktoren mit innenliegenden Kühlsystemen oder mit Mantelkühlem oder mit einem äußeren Umlauf mit Wärmetauschern können für das erfindungsgemäße Verfahren eingesetzt werden.

Diese für die erfindungsgemäße Anlage vorteilhaften Konstruktionsmerkmale werden zweckmäßigerweise auch bei Einsatz eines zonenweise unterteilten Einzelreaktors realisiert.

Es ist daher sinnvoll, den Reaktor so zu gestalten, daß er zonenweise geheizt und/oder gekühlt werden kann.

Ferner weist ein solcher Einzelreaktor vorteilhafterweise mehrere, über seine Länge verteilte, den einzelnen Reaktionszonen zugeordnete Zuführungen für Reaktanden und Abführungen für Austräge sowie gegebenenfalls den Reaktionszonen zugeordnete heiz- und/oder kühlbare Außenkreisläufe auf.

Die in dem Reaktor bzw. den Reaktorelementen eingebauten Vorrichtungen zur Feinverteilung der Reaktanten sind beispielsweise Siebböden, Fritten oder Jetdüsen. Ferner ist es vorteilhaft, wenn die Reaktorelemente bzw. der zonenweise unterteilte Reaktor strömungsbrechende Einbauten und/oder Rührvorrichtungen aufweisen.

Die Destillationsvorrichtung B 1 der erfindungsgemäßen Produktionsanlage hat die Aufgabe, Methylformiat aus dem Feedstrom heraus zu destillieren, d.h. durch selektives Verdampfen abzutrennen. Zur Herstellung eines für die Ameisensäureherstellung geeigneten Methylformiats genügt zum Abdestillieren in der Regel eine einfache Abtriebskolonne. Es ist jedoch auch möglich, die Kolonne mit einem Verstärkungsteil zu versehen, um einen Methylformiatstrom höherer Reinheit über Kopf abziehen zu können. Die Kolonne wird dann unter Rücklauf als Rektifikationskolonne betrieben.

Eine solche Kolonne besteht im wesentlichen aus einer erforderlichenfalls zonenweise beheiz- oder kühlbaren, mit Trennböden versehenen rohrförmigen Hohlkörper mit einer in geeigneter Position angebrachten Zuführung für den entgasten Reaktoraustrag, Abführungen zum Abzug von Destillat über Kopf und Katalysator und Katalysatorabbauprodukte enthaltendes Methanol aus dem Sumpf, sowie gegebenenfalls weiteren Zuführungen für flüssige oder gasförmige Wärmeträger.

Zweckmäßigerweise wird hier eine Kolonne eingesetzt, deren Trennwirkung genügt, um aus dem entgasten Reaktoraustrag über Kopf Methylformiat der benötigten Reinheit abzuziehen.

In der Regel wird die Kolonne auf eine Gewinnung von 85 bis 90 gew.-%igem Methylformiat (Rest = Methanol) eingerichtet. Vorteilhafterweise findet das Abdestillieren des erhaltenen Methylformiats aus dem Reaktionsgemisch, in einer einfachen Abtriebskolonne ohne Trennböden derart statt, daß man eine Aufkonzentrierung von 70 bis 80 Gew.-% - der Konzentration im Reaktionsgemisch - auf 85 bis 90 Gew.-% erhält. Dies erfordert gegenüber einer Reindestillation zu 95 bis 97 %igem Methylformiat erheblich weniger Energie. Erfindungsgemäß hergestelltes Methylformiat mit einem Reingehalt von beispielsweise 85 bis 90 Gew.% kann technisch vielseitig verwendet werden. Insbesondere kann es direkt für die Hydrolyse zur Herstellung von Ameisensäure eingesetzt werden.

Die Entsalzungsvorrichtung B3 besteht im wesentlichen aus
1) einer erforderlichenfalls zonenweise beheiz- oder kühlbaren, ggf. mit Trennböden versehenen Rektifizierkolonne, deren Trennwirkung ausreicht, um über Kopf Methanol mit weniger als 100 ppm, vorzugsweise weniger als 30 ppm. insbesondere mit etwa 5 bis 15 ppm Wassergehalt abzuziehen,
2) einer in geeigneter Position angebrachten Zuführung für Restkatalysator und Katalysatorabbauprodukte enthaltendes Methanol,
3) einer in geeigneter Position angebrachten Zuführung für Heißwasser und/oder Wasserdampf, und
4) Abführungen zum Abzug von Destillat über Kopf, und Katalysatorabbauprodukte enthaltendes Wasser aus dem Sumpf.

In der Regel genügt für diesen Zweck eine einfache Abtriebskolonne.

Rektifizierkolonnen, wie sie beispielsweise in Ullmann's Encyclopedia of Industrial Chemistry VCH (1988), Vol. B3, 4-62 bis 4-65 beschriebenen sind, dienen dazu, Gemische von mehreren Komponenten mit unterschiedlichen Dampfdrucken zu trennen. Diesen bekannten Rektifizierkolonnen werden an einer geeigneten Stelle die zu trennenden Gemische zugeführt und sowohl über Kopf als auch aus dem Sumpf werden wieder Gemische der gleichen Komponenten abgezogen, die sich - abgesehen von Nebenprodukten, die durch die Hitzeeinwirkung während der Destillation gebildet werden - voneinander und von dem zugeführten Feed lediglich in Bezug auf ihr Mischungsverhältnis unterscheiden.
Erreicht wird diese Stofftrennung durch eine in allen Ebenen (Böden) längs eines Temperaturgradienten erzwungene Einstellung des Dampfdruckgleichgewichts zwischen Flüssigkeit und Dampf eines Gemisches der gleichen Komponenten.

Der der Entsalzungsvorrichtung zugeführte Dampf oder das Heißwasser wird im oder vorzugsweise unterhalb des Bereichs der Zuführung für das Restkatalysator und Katalysatorabbauprodukte enthaltende Methanol, insbesondere im Sumpfbereich, in die Vorrichtung eingeleitet.

Das über Kopf der Entsalzungsvorrichtung abgezogene Methanol kann kondensiert und entweder in die Synthesegruppe zurückgeführt oder unter einem solchen Druck verdampft werden, daß es als gasförmiger Wärmeträger in die Destillationsvorrichtung eingespeist werden kann. Dazu wäre dann ein weiterer Verdampfer am Sumpf der Destillationskolonne erforderlich.

Besonders vorteilhaft und ökonomisch ist es allerdings, wenn die Destillationsvorrichtung und die Entsalzungsvorrichtung im direkten Wärmeverbund arbeiten, indem der Kopfdruck der Entsalzungsvorrichtung so hoch eingestellt wird, daß der daraus abgeleitete Methanoldampf direkt in die Destillationsvorrichtung als gasförmiger Wärmeträger eingeleitet werden kann. Es ist prinzipiell auch möglich, durch eine äquivalente Verbundkonstruktion die Destillations- und die Entsalzungskolonne in eine Kolonne zusammenzufassen, die mit Seiten-Abzügen bzw. -Zuführungen für Flüssigphasen versehen ist.

Eine weitere, besonders ökonomische Ausfürungsform der vorliegenden Erfindung besteht darin, daß man die Aufarbeitungsaggregate, insbesondere die Entsalzungsvorrichtung mit der Abwärme des Reaktors betreibt, gegebenenfalls unter Einschaltung einer Wärmepumpe.
Ein weiterer Gegenstand der vorliegenden Erfindung ist die oben bereits beschriebene feststofffrei arbeitende Entsalzungsvorrichtung, im Wesentlichen bestehend aus einer erforderlichenfalls zonenweise beheiz- oder kühlbaren, gegebenenfalls mit Trennböden versehenen Rektifizierkolonne mit einer in geeigneter Position angebrachten Zuführungen für Restkatalysator und Katalysatorabbauprodukte enthaltendes Methanol und für Heißwasser und/oder Wasserdampf, Abführungen zum Abzug von Destillat über Kopf und Katalysatorabbauprodukte enthaltendes Wasser aus dem Sumpf, deren Trennwirkung ausreicht, um über Kopf Methanol mit weniger als 100 ppm, vorzugsweise weniger als 30 ppm insbesondere mit etwa 5 bis 15 ppm Wassergehalt abzuziehen.

In einer bevorzugten Ausführungsform wird der der Entsalzungsvorrichtung zugeführte Dampf oder das Heißwasser im, oder vorzugsweise unterhalb, des Bereichs der Zuführung für das Restkatalysator und Katalysatorabbauprodukte enthaltende Methanol, insbesondere im Sumpfbereich, in die Vorrichtung eingeleitet.

Ein weiterer Gegenstand der vorliegenden Erfindung ist die beschriebene Kombination von Destillations- und Entsalzungsvorrichtung, wobei der über Kopf der Entsalzungsvorrichtung abgehende Methanoldampf als Wärmeträger in die Destillationsvorrichtung eingeleitet wird. Die folgenden Ausführungsbeispiele veranschaulichen das erfindungsgemäße Verfahren und die zu seiner Ausführung eingesetzte erfindungsgemäße Vorrichtung.

### Beispiel 1

Drei senkrecht stehende Rohrreaktoren mit einem Volumen von je 2 l, je einem Außenkreislauf mit Wärmetauscher, Zuführungen für Flüssigphase und Abführungen für Gasphase im Kopfbereich und Abführungen für Flüssigphase und Jet-Düsen-Zuführungen für Gasphase im Bodenbereich, wurden übereinanderliegend so in Serie geschaltet, daß am Boden des untersten Reaktors eingeführte Gasphase nacheinander alle drei Reaktoren aufsteigend und im Kopfbereich des obersten Reaktors zugeführte Flüssigphase alle drei Reaktoren nacheinander absteigend durchströmen kann.

In dieser Syntheseanordnung wurden 12 Versuche ausgeführt, wobei der Reaktorkaskade vom Kopf her Methanol und Katalysator in Form einer methanolischen Alkalimethylatlösung und durch die Jetdüsen von unten CO in den in der Tabelle 1 angegebenen Mengen zugeführt wurde. Der Druck und die Temperatur in den Reaktoren wurden auf die in der Tabelle 1 ebenfalls angegebenen Werte eingestellt.

Der am Boden abgezogene Austrag aus der Reaktorkaskade wurde in einer Entspannungsvorrichtung auf einen Druck von 15 bar entspannt, das dabei auftretende Sprudelgas zur Abscheidung von gasförmig darin mitgeführtem Methanol und Methylformiat in einem zweistufigen Kühler auf Raumtemperatur abgekühlt. Die verbleibende Gasphase wird in die Reaktorkaskade zurückgeführt, die vereinigten Flüssigphasen wurden gaschromatographisch und naßanalytisch auf ihren Methylformiatgehalt untersucht.

Die 4 letzten Spalten der Tabelle 1 zeigen die erhaltenen Versuchsergebnisse.

Die in der Tabelle benutzten Abkürzungen haben folgende Bedeutungen:

RZA steht für die Produktionskapazität, bezogen auf das Gesamtvolumen aller eingesetzten Reaktoren,

NaOMe bedeutet Natriummethylat, KOMe bedeutet Kaliummethylat und MeFo steht für Methylformiat.

Die angegebenen Umsätze sind bezogen auf eingesetztes Methanol

Zur weiteren Aufarbeitung des Reaktoraustrags wird dieser einer einfachen Abtriebskolonne zugeführt, worin bei einem Druck von ca. 1,8 bar durch eingeleiteten Methanoldampf das Methylformiat weitgehend ausgetrieben wird.

Das aus dem Sumpf dieser Kolonne abgezogene Gemisch aus Methanol und verbrauchtem und unverbrauchtem Katalysator wird einem Stromteiler zugeführt aus welchem 1/3 des Gemisches in die Reaktorkaskade zurückgeführt und 2/3 in die Entsalzungskolonne eingespeist werden. In der Entsalzungskolonne erfolgt bei einem Innendruck von ca 2 bar eine Behandlung der zugeführten, Katalysator und Salze enthaltenden methanolischen Lösung mit Wasserdampf von 4 bar. Die Menge des zugeführten Wasserdampfs wird so bemessen, daß am Kopf der Kolonne praktisch wasserfreier Methanoldampf abgenommen werden kann, der zweckmäßigerweise als Wärmeträger der Destillationskolonne direkt zugeführt wird. Aus dem Sumpf der Entsalzungskolonne wird eine wäßrige Alkalihydroxid-und Alkaliformiatlösung abgezogen.

### Beispiel 2

Zwei Rohrreaktoren von 200 cm Länge und 4,5 cm Innendurchmesser (Volumen = 3,15 1) mit innenliegendem Wärmetauscherrohr, Zuführungen für Flüssig- und Gasphase im Bodenbereich und Abführungen für Flüssig- und Gasphase im Kopfbereich, wobei die Zuführungen für die Gasphase als Jet-Düsen ausgebildet sind, wurden übereinanderliegend so in Serie geschaltet, daß am Boden des untersten Reaktors eingeführte Flüssig- und Gasphase nacheinander alle drei Reaktoren im Gleichstrom aufsteigend durchströmen kann.

In dieser Syntheseanordnung wurden 19 Versuche ausgeführt, wobei der Reaktorkaskade vom Boden her Methanol, Katalysator in Form einer methanolischen Alkalimethylatlösung und durch die Jetdüsen CO in den in der Tabelle 2 angegebenen Mengen zugeführt wurde. Der Druck und die Temperatur in den Reaktoren wurden auf die in der Tabelle 2 ebenfalls angegebenen Werte eingestellt.

Die 4 letzten Spalten der Tabelle 2 zeigen die erhaltenen Versuchsergebnisse.

Die weitere Aufarbeitung des Reaktoraustrags erfolgt wie in Beispiel 1 beschrieben.

**Tabelle 1, Versuche im Gegenstrom.**

| **Beispiel Nr.** | **CO[l/h]** | **MeOH [kg/h]** | **Katalysator** | | **Druck [bar]** | **Temperatur [°C],** **gemessen in der Mitte der Reaktoren R** | | | **MeFo im Austrag, [Gew.-%]** | **Salzausfall [ja/nein]** | **Umsatz ² [%]** | **RZA [kg/m³/h]** |
|---|---|---|---|---|---|---|---|---|---|---|---|---|
| | | | **Art** | **[Gew.-%]¹** | | **R1** | **R2** | **R3** | | | | |
| 1 | 1110 | 2,5 NaOMe | | 0,20 | 109 | 90 | 90 | 90 | 75 | Nein | 61,5 | 520 |
| 2 | 1790 | 2,9 NaOMe | | 0,20 | 136 | 90 | 90 | 88 | 80 | Nein | 68,1 | 750 |
| 3 | 1120 | 2,3 NaOMe | | 0,30 | 95 | 90 | 90 | 85 | 74 | Nein | 60,3 | 580 |
| 4 | 1540 | 3,3 NaOMe | | 0,30 | 111 | 90 | 90 | 84 | 74 | Nein | 60,3 | 830 |
| 5 | 970 | 2,6 NaOMe | | 0,20 | 97 | 100 | 100 | 90 | 67 | Nein | 52,0 | 550 |
| 6 | 1640 | 3,5 NaOMe | | 0,20 | 117 | 100 | 100 | 90 | 73 | Nein | 59,1 | 880 |
| 7 | 1600 | 3,5 NaOMe | | 0,30 | 97 | 100 | 100 | 95 | 68 | Nein | 53,1 | 790 |
| 8 | 720 | 1,5 KOMe | | 0,20 | 112 | 90 | 90 | 76 | 73 | Nein | 59,1 | 370 |
| 9 | 950 | 2,0 KOMe | | 0,20 | 126 | 90 | 90 | 72 | 75 | Nein | 61,5 | 520 |
| 10 | 950 | 2,0 KOMe | | 0,26 | 105 | 90 | 90 | 82 | 73 | Nein | 59,1 | 480 |
| 11 | 970 | 2,5 KOMe | | 0,26 | 95 | 100 | 100 | 91 | 63 | Nein | 47,6 | 520 |
| 12 | 1590 | 3,5 KOMe | | 0,26 | 116 | 100 | 100 | 79 | 69 | Nein | 54,3 | 790 |

| | | | | | | | | | | | | |
|---|---|---|---|---|---|---|---|---|---|---|---|---|
| 1) Bezogen auf das Gewicht des flüssigen Feeds 2) Bezogen auf eingesetztes Methanol | | | | | | | | | | | | |

**Tabelle 2, Versuche im Gleichstrom**

| **Beispiel Nr.** | **CO [l/h]** | **MeOH [kg/h]** | **Katalysator** | | **Druck [bar]** | **Temperatur [°C], gemessen in der Mitte der Reaktoren R** | | **MeFo im Austrag, [Gew.-%]** | **Salzausfall [ja/nein]** | **Umsatz ² [%]** | **RZA [kg/m³/h]** |
|---|---|---|---|---|---|---|---|---|---|---|---|
| | | | **Art** | **[Gew.-%]¹** | | **R1** | **R2** | | | | |
| 1 | 1864 | 2,99 | NaOMe | 0,18 | 130 | 100 | 100 | 76,9 | Nein | 64,0 | 560 |
| 2 | 1836 | 2,97 | NaOMe | 0,20 | 110 | 100 | 110 | 69,4 | Nein | 54,8 | 470 |
| 3 | 1952 | 2,91 | NaOMe | 0,21 | 120 | 100 | 110 | 73,5 | Nein | 59,7 | 510 |
| 4 | 2101 | 2,97 | NaOMe | 0,20 | 140 | 100 | 110 | 79,0 | Nein | 66,7 | 580 |
| 5 | 2186 | 3,01 | NaOMe | 0,20 | 150 | 100 | 110 | 81,0 | Nein | 69,5 | 610 |
| 6 | 1798 | 2,99 | NaOMe | 0,20 | 140 | 90 | 100 | 80,2 | Nein | 68,4 | 600 |
| 7 | 1721 | 2,59 | NaOMe | 0,25 | 140 | 90 | 100 | 86,8 | Nein | 77,8 | 590 |
| 8 | 1428 | 2,99 | NaOMe | 0,12 | 140 | 100 | 110 | 70,6 | Nein | 56,2 | 480 |
| 9 | 2031 | 4,00 | NaOMe | 0,21 | 140 | 100 | 110 | 80,2 | Nein | 68,4 | 790 |
| 10 | 2330 | 4,99 | NaOMe | 0,21 | 140 | 100 | 110 | 72,3 | Nein | 58,2 | 840 |
| 11 | 1851 | 3,11 | NaOMe | 0,30 | 140 | 100 | 110 | 80,5 | Nein | 68,8 | 620 |
| 12 | 2430 | 5,89 | NaOMe | 0,21 | 140 | 100 | 110 | 66,1 | Nein | 51,0 | 870 |
| 13 | 2064 | 4,19 | NaOMe | 0,19 | 140 | 100 | 110 | 73,8 | Nein | 60,0 | 720 |
| 14 | 2484 | 4,90 | NaOMe | 0,32 | 140 | 100 | 110 | 74,7 | Nein | 61,2 | 860 |
| 15 | 2722 | 6,01 | NaOMe | 0,31 | 150 | 100 | 110 | 71,3 | Nein | 57,0 | 990 |
| 16 | 1706 | 3,56 | NaOMe | 0,12 | 160 | 100 | 110 | 71,1 | Nein | 56,8 | 590 |
| 17 | 1857 | 3,55 | NaOMe | 0,15 | 160 | 100 | 110 | 75,8 | Nein | 62,6 | 650 |
| 18 | 2002 | 4,09 | NaOMe | 0,19 | 140 | 100 | 95 | 73,7 | Nein | 59,9 | 710 |
| 19 | 2564 | 3,77 | NaOMe | 0,21 | 140 | 95 | 110 | 78,2 | Nein | 65,7 | 730 |

## Patentansprüche

1. Verfahren zur Herstellung von Methylformiat durch Umsetzung von überschüssigem Methanol mit Kohlenmonoxid unter erhöhtem Druck und erhöhter Temperatur in Gegenwart von Alkali- oder Erdalkalimethylat als Katalysator in einem druckfesten Reaktor, Abtrennung des gebildeten Methylformiats aus dem Reaktoraustrag und Rückführung der im wesentlichen methylformiatfreien Flüssigphase in den Reaktor, wobei ein Teil der rückzuführenden Flüssigphase ausgeschleust und frische Katalysatorlösung zugeführt wird, **dadurch gekennzeichnet, daß**
die Umsetzung in einer Kaskade aus mindestens 2 Reaktorelementen
bei einer Temperatur von 80 bis 120°C,
unter einem Kohlenmonoxid-Druck von 90 bis 180 bar,
in Gegenwart von 0,05 bis 0,5 Gew.-%, bezogen auf das Gewicht des flüssigen Feeds, eines Alkali- oder Erdalkalialkoholats ausgeführt wird,
daß das Verhältnis der in der Zeiteinheit zugeführten Mengen der Ausgangsmaterialien, die Reaktionstemperatur, der Druck und die Verweilzeit der Reaktanten in den Reaktorelementen so eingestellt werden, daß mindestens soviel des Methanols unumgesetzt bleibt, daß sowohl der verwendete Katalysator als auch dessen Abbauprodukte unter den Reaktionsbedingungen im Reaktor und im frischen Reaktoraustrag praktisch vollständig gelöst bleiben,
daß der gesamte Reaktoraustrag einer Destillationsvorrichtung zugeführt wird, in der im wesentlichen das Methylformiat aus der Reaktionsmischung ausgetrieben wird,
daß ein Teil TR der verbleibenden Flüssigphase in den Reaktor zurückgeführt und ein Teil TA von 20 bis 80 % der verbleibenden Flüssigphase ausgeschleust wird,
daß dem ausgeschleusten Teil in einer Entsalzungsvorrichtung Restkatalysator und Katalysator-Abbauprodukte feststofffrei entzogen werden, und das verbleibende Methanol unmittelbar oder mittelbar in den Reaktor zurückgeführt wird.

2. Verfahren gemäß Anspruch 1, **dadurch gekennzeichnet, daß** zur Durch-führung des Verfahrens 2 bis 5 Reaktorelemente eingesetzt werden.

3. Verfahren gemäß den Ansprüchen 1 und 2, **dadurch gekennzeichnet, daß** dem ausgeschleusten Anteil TA der nach der Abtrennung des Methylformiats verbleibenden Flüssigphase, bestehend im wesentlichen aus Katalysator und Katalysatorabbauprodukte enthaltendem Methanol in der Entsalzungsvorrichtung soviel Wasserdampf und/oder Heißwasser und gegebenenfalls zusätzliche Wärme zugeführt wird, daß das Methanol im wesentlichen vollständig verdampft, und eine wäßrige Lösung der Katalysatorabbauprodukte erhalten wird.

4. Verfahren gemäß den Ansprüchen 1 bis 3, **dadurch gekennzeichnet, daß** die Entsalzungsvorrichtung mit der Destillations-vorrichtung in einem Wärmeverbund betrieben wird, wobei der über Kopf der Entsalzungsvorrichtung entweichende Methanoldampf der Destillations-vorrichtung zugeführt wird.

5. Anlage zur Produktion von Methylformiat nach dem Verfahren des Anspruchs 1, mit
A) einer Synthesegruppe im wesentlichen bestehend aus
A1) einem Reaktor mit mindestens zwei separat heiz- und kühlbaren Reaktorelementen mit Zuführungen für frisches Methanol, rückgeführtes katalysatorhaltiges Methanol, für frische methanolische Katalysatorlösung und für ein Kohlenmonoxid enthaltendes Gasgemisch, mindestens je einer Ableitung für den Reaktoraustrag und Restgas, Vorrichtungen zur Erzeugung und Aufrechterhaltung einer Feinverteilung des Gasstromes in dem Flüssigkeitsstrom Meßvorrichtungen zur Temperatur- und Druckkontrolle,
A2) einer mit Kühlelementen versehenen Entspannungsvorrichtung zur Entspannung des Reaktoraustrags auf den Aufarbeitungsdruck mit einer Zuführung für den Reaktoraustrag und Ableitungen für Restgas und Flüssigphase
B) einer Aufarbeitungsgruppe im wesentlichen bestehend aus
B1) einer Destillationsvorrichtung zur Abtrennung von im wesentlichen Methylformiat aus der Flüssigphase des Reaktoraustrags, mit einer Zu-führung für die Flüssigphase und Ableitungen für im wesentlichen Methyl-formiat und für verbleibendes katalysatorhaltiges Methanol,
B2) einem regelbaren Stromteiler für die Aufteilung des die Destillationsvorrichtung verlassenden Restkatalysator und Katalysatorabbauprodukte enthaltenden Methanol-Stromes in die Anteile TR und TA,
B3) einer gegebenenfalls mit Heiz- und Kühlelementen versehenen, feststofffrei arbeitenden Entsalzungsvorrichtung, mit Einlässen für Restkatalysator und Katalysatorabbauprodukte enthaltendes Methanol und Heißwasser oder Wasserdampf und Auslässen für Methanoldampf und wäßrige Salzlösung,
C) Verbindungsleitungen und erforderlichenfalls Pumpen für eine zweckentsprechende Förderung der Reaktionsteilnehmer und -produkte zwischen den Elementen der Anlagenteile A und B und Zuführungen für Ausgangsmaterialien und Ableitungen für Methylformiat und Abgas.

6. Anlage gemäß Anspruch 5, **dadurch gekennzeichnet, daß** als Destillationsvorrichtung B1 eine Kolonne eingesetzt wird, deren Trennwirkung genügt, um aus dem entgasten Reaktoraustrag über Kopf Methylformiat der benötigten Reinheit abzuziehen.

7. Anlage gemäß den Ansprüchen 5 und 6, **dadurch gekennzeichnet, daß** die Entsalzungsvorrichtung B3 im wesentlichen aus einer erforderlichenfalls zonenweise beheiz- oder kühlbaren, ggf. mit Trennböden versehenen Rektifizierkolonne besteht, mit einer in geeigneter Position angebrachten Zuführungen für Restkatalysator und Katalysator-abbauprodukte enthaltendes Methanol und für Heißwasser und/oder Wasserdampf, Abführungen zum Abzug von Destillat über Kopf, und Katalysatorabbauprodukte enthaltendes Wasser aus dem Sumpf, deren Trennwirkung ausreicht, um über Kopf Methanol mit weniger als 100 ppm, vorzugsweise weniger als 30 ppm insbesondere mit etwa 5 bis 15 ppm Wassergehalt abzuziehen.

8. Verfahren zum feststofffreien Entsalzen einer Flüssigphase, bestehend im wesentlichen aus Methanol, einem Alkali- und/oder Erdalkalialkoholate umfassenden Katalysator und Katalysator-Abbauprodukten
**dadurch gekennzeichnet,**
**daß** der Flüssigphase in einer Entsalzungsvorrichtung soviel Wasserdampf und/oder Heißwasser und gegebenenfalls zusätzlich Wärme zugeführt wird, daß das Methanol im wesentlichen vollständig verdampft und der Katalysator und seine Abbauprodukte sicher in in wäßriger Lösung gehalten werden.

9. Verfahren gemäß Anspruch 8, **dadurch gekennzeichnet, daß** soviel Wasserdampf und/oder Heißwasser und gegebenenfalls zusätzlich Wärme zugeführt wird, daß eine 5 bis 20 %ige wäßrige Lösung der Katalysator-Abbauprodukte gebildet wird.

10. Verfahren gemäß Anspruch 8, **dadurch gekennzeichnet, daß** der Wasserdampf mit einem Druck von 2 bis 6 bar zugeführt wird.

## Claims

1. A process for preparing methyl formate by reacting excess methanol with carbon monoxide under superatmospheric pressure and at elevated temperature in the presence of alkali metal methoxide or alkaline earth metal methoxide as catalyst in a pressure-rated reactor, separating the methyl formate formed from the reaction product and recirculating the liquid phase which is essentially free of methyl formate to the reactor, with part of the liquid phase to be recirculated being discharged and fresh catalyst solution being fed in, wherein
the reaction is carried out in a cascade comprising at least two reactor elements
at from 80 to 120°C,
under a carbon monoxide pressure of from 90 to 180 bar,
in the presence of from 0.05 to 0.5% by weight, based on the weight of the liquid feed, of an alkali metal alkoxide or alkaline earth metal alkoxide,
the ratio of the amounts of starting materials fed in per unit time, the reaction temperature, the pressure and the residence time of the reactants in the reactor elements are set so that at least that amount of methanol required to keep both the catalyst used and its degradation products virtually completely dissolved under the reaction conditions in the reactor and in the fresh reaction product remains unreacted,
the total output from the reactor is passed to a stripping apparatus in which essentially the methyl formate is stripped from the reaction mixture,
a part TR of the remaining liquid phase is recirculated to the reactor and a part TA consisting of from 20 to 80% of the remaining liquid phase is discharged,
and residual catalyst and catalyst degradation products are removed solids-free from the discharged part in a desalting apparatus and the remaining methanol is returned directly or indirectly to the reactor.

2. The process according to claim 1 carried out using from 2 to 5 reactor elements.

3. The process according to claims 1 and 2, wherein steam and/or hot water and, if appropriate, additional heat are fed to the discharged part TA of the liquid phase remaining after separating off the methyl formate, consisting essentially of methanol comprising catalyst and catalyst degradation products, in the desalting apparatus in such amounts that the methanol is essentially completely vaporized and an aqueous solution of the catalyst degradation products is obtained.

4. The process according to claims 1 to 3, wherein the desalting apparatus is operated as an integrated heat system with the distillation apparatus and the methanol vapor leaving the top of the desalting apparatus is fed to the distillation apparatus.

5. A plant for producing methyl formate by the process of claim 1, comprising
A) a synthesis group consisting essentially of
A1) a reactor having at least two separately heatable and coolable reactor elements with feed lines for fresh methanol, recirculated catalyst-containing methanol for fresh methanolic catalyst solution and for a gas mixture comprising carbon monoxide, at least one outlet line each for the reaction product and residual gas, devices for generating and maintaining a fine dispersion of the gas stream in the liquid stream, and instrumentation for monitoring temperature and pressure,
A2) a depressurization apparatus provided with cooling elements for depressurizing the reaction product to the work-up pressure and provided with a feed line for the reaction product and outlet lines for residual gas and liquid phase,
B) a work-up group consisting essentially of
B1) a distillation apparatus for separating essentially methyl formate from the liquid phase of the reaction product provided with a feed line for the liquid phase and outlet lines for essentially methyl formate and for remaining catalyst-containing methanol,
B2) an adjustable stream divider for dividing the methanol stream comprising residual catalyst and catalyst degradation products leaving the distillation apparatus into the substreams TR and TA,
B3) a desalting apparatus which may, if appropriate, be provided with heating and cooling elements and operates in a solids-free manner, provided with inlets for methanol comprising residual catalyst and catalyst degradation products and for hot water or steam and outlets for methanol vapor and for aqueous salt solution,
C) connection lines and, if required, pumps for appropriate transport of reaction participants and products between the elements of the plant sections A and B and feed lines for starting materials and outlet lines for methyl formate and waste gas.

6. The plant according to claim 5, wherein the distillation apparatus B1 used is a column whose separation efficiency is sufficient for methyl formate of the required purity to be taken off from the degassed reaction product via the top.

7. The plant according to claims 5 and 6, wherein the desalting apparatus B3 consists essentially of a rectification column which can be heated or cooled in zones if necessary, may, if appropriate, be provided with separation plates and is equipped in suitable positions with feed lines for methanol comprising residual catalyst and catalyst degradation products and for hot water and/or steam, outlet lines for taking off distillate at the top and water comprising catalyst degradation products from the bottom, and whose separation efficiency is sufficient to take off methanol containing less than 100 ppm, preferably less than 30 ppm, in particular from about 5 to 15 ppm, of water at the top.

8. A process for the solids-free desalting of a liquid phase consisting essentially of methanol, a catalyst comprising alkali metal alkoxides and/or alkaline earth metal alkoxides and catalyst degradation products, which comprises introducing steam and/or hot water and, if appropriate, additional heat into the liquid phase in a desalting apparatus in such quantities that the methanol is essentially completely vaporized and the catalyst and its degradation products are reliably kept in aqueous solution.

9. The process according to claim 8, wherein steam and/or hot water and, if appropriate, additional heat are introduced in such quantities that a from 5 to 20% strength aqueous solution of the catalyst degradation products is formed.

10. The process according to claim 8, wherein the steam is introduced at a pressure of from 2 to 6 bar.

## Revendications

1. Procédé de préparation de méthylformiate par transformation de méthanol excédentaire avec du monoxyde de carbone sous pression accrue et température augmentée en présence de méthylate alcalin ou alcalino-terreux comme catalyseur dans un réacteur résistant à la pression, séparation du méthylformiate formé hors de la sortie du réacteur et recyclage de la phase liquide essentiellement exempte de méthylformiate dans le réacteur, une partie de la phase liquide à recycler étant évacuée et une solution fraîche de catalyseur étant amenée, **caractérisé en ce que**
la transformation s'effectue dans une cascade d'au moins 2 éléments de réacteur à une température de 80 à 120°C,
à une pression du monoxyde de carbone de 90 à 180 bars, en présence de 0,05 à 0,5% en poids, sur base du poids de la charge liquide, d'un alcoolate alcalin ou alcalino-terreux,
le rapport des quantités amenées dans l'unité de temps des matières de départ, la température réactionnelle, la pression et la durée de séjour des réactants dans les éléments de réacteur sont réglés de telle sorte qu'il reste au moins suffisamment de méthanol non réagi pour qu'autant le catalyseur utilisé que ses produits de décomposition, dans les conditions réactionnelles dans le réacteur et dans la sortie fraîche du réacteur restent dissous presque entièrement,
la sortie du réacteur dans son entier est amenée à une installation de distillation dans laquelle le méthylformiate essentiellement est chassé du mélange réactionnel,
une partie TR de la phase liquide restante est recyclée dans le réacteur et une partie TA de 20 à 80% de la phase liquide restante est évacuée,
le catalyseur résiduel et les produits de décomposition du catalyseur sont soutirés sans solide de la partie évacuée dans une installation de dessalement et le méthanol restant est directement ou indirectement recyclé dans le réacteur.

2. Procédé selon la revendication 1, **caractérisé en ce que** pour l'exécution du procédé, on utilise 2 à 5 éléments de réacteur.

3. Procédé selon les revendications 1 et 2, **caractérisé en ce qu'**on amène, à l'installation de dessalement et à la partie évacuée TA de la phase liquide restante après la séparation du méthylformiate, essentiellement composée de méthanol contenant un catalyseur et des produits de décomposition du catalyseur, suffisamment de vapeur d'eau et/ou d'eau chaude et le cas échéant de chaleur supplémentaire pour évaporer le méthanol quasiment entièrement et on obtient une solution aqueuse des produits de décomposition du catalyseur.

4. Procédé selon les revendications 1 à 3, **caractérisé en ce que** l'installation de dessalement fonctionne avec l'installation de distillation en raccord de chaleur, la vapeur de méthanol ramollissante de l'installation de distillation étant amenée par la tête de l'installation de dessalement.

5. Installation de produit de méthylformiate selon le procédé de la revendication 1, avec :
A) un groupe de synthèse composé essentiellement de :
A1) un réacteur avec au moins deux éléments de réacteur séparément chauffants et réfrigérants avec des conduites d'amenée pour du méthanol frais, du méthanol contenant un catalyseur recyclé, une solution de catalyseur fraîche méthanolique et un mélange gazeux contenant du monoxyde de carbone, au moins chacun une dérivation pour la sortie de réacteur et le gaz résiduel, des dispositifs de production et de maintien d'une répartition fine du courant de gaz dans le courant de liquide, des dispositifs de mesure de température et de contrôle de la pression,
A2) un dispositif de dilatation muni d'éléments refroidisseurs pour la dilatation de la sortie de réacteur à la pression de traitement avec une conduite pour la sortie de réacteur et des dérivations pour le gaz résiduel et la phase liquide,
B) un groupe de traitement composé essentiellement de :
B1) d'un dispositif de distillation pour la séparation en grande partie du méthylformiate hors de la phase liquide de la sortie de réacteur, avec une amenée de la phase liquide et des dérivations pour essentiellement du méthylformiate et pour le méthanol restant contenant le catalyseur,
B2) un diviseur de courant réglable pour la répartition du courant de méthanol contenant de catalyseur résiduel quittant le dispositif de distillation et les produits de décomposition du catalyseur dans les parties TR et TA,
B3) un dispositif de dessalement travaillant sans solides muni éventuellement d'éléments de chauffage et de refroidissement, avec des admissions pour le catalyseur résiduel et le méthanol contenant les produits de décomposition du catalyseur et de l'eau chaude ou de la vapeur d'eau et des sorties pour la vapeur de méthanol et la solution saline aqueuse,
C) des conduites de liaison et des pompes en cas de besoin pour le transport selon les buts des constituants et produits réactionnels entre les éléments de la partie A et B des installations et des conduites d'amenée pour les matières de départ et des dérivations pour le méthylformiate et le gaz d'échappement.

6. Installation selon la revendication 5, **caractérisée en ce qu'**on utilise comme dispositif de distillation B1 une colonne dont l'action de séparation suffit pour soutirer de la sortie du réacteur dégazée et par la tête, du méthylformiate de la pureté voulue.

7. Installation selon les revendications 5 et 6, **caractérisée en ce que** le dispositif de dessalement B3 est essentiellement constitué d'une colonne de rectification chauffante ou réfrigérante, si nécessaire en zone, ou, selon le cas, munie de plateaux de séparation, avec une conduite agencée en position adéquate pour le catalyseur résiduel et/ou le méthanol contenant des produits de décomposition du catalyseur et pour l'eau chaude et/ou la vapeur d'eau, des conduites d'évacuation pour le soutirage du distillat par la tête, et l'eau contenant les produits de décomposition du catalyseur du bas dont l'action de séparation suffit pour soutirer par la tête le méthanol avec moins de 100 ppm, de préférence moins de 30 ppm, en particulier avec environ 5 à 15 ppm de teneur en eau.

8. Procédé de dessalement sans solide d'une phase liquide composée essentiellement de méthanol, d'un catalyseur comprenant des alcoolates alcalins et/ou alcalino-terreux et des produits de décomposition de catalyseur
**caractérisé en ce que**
on amène à la phase liquide dans un dispositif de dessalement autant de vapeur d'eau et/ou d'eau chaude et le cas échéant de chaleur supplémentaire que nécessaire pour que le méthanol s'évapore quasiment totalement et que le catalyseur et ses produits de décomposition soient gardés sûrement dans la solution aqueuse.

9. Procédé selon la revendication 8, **caractérisé en ce qu'**on amène suffisamment de vapeur d'eau et/ou d'eau chaude et le cas échéant de chaleur supplémentaire pour qu'il se forme une solution aqueuse entre 5 et 20% des produits de décomposition du catalyseur.

10. Procédé selon la revendication 8, **caractérisé en ce que** la vapeur d'eau est amenée à une pression comprise entre 2 et 6 bars.
